# EUROPÄISCHE PATENTSCHRIFT

(11) **EP 3 364 960 B1**
(45) Veröffentlichungstag und Bekanntmachung des Hinweises auf die Patenterteilung: **20.03.2024**
(21) Anmeldenummer: 16747738.9
(22) Anmeldetag: 08.07.2016
(51) Int. Cl.: A61K 31/19, A61P 37/02, A61K 9/20, A61K 9/48, A61K 31/215, A61K 31/25

(54) **MITTEL ZUR UNTERSTÜTZENDEN IMMUNMODULATION ENTHALTEND PROPIONSÄURE UND EINEN FUMARSÄUREESTER**
AGENT FOR SUPPORTIVE IMMUNOMODULATION CONTAINING PROPIONIC ACID AND FUMARIC ACID ESTER
AGENT POUR LE SOUTIEN DE L'IMMUNOMODULATION CONTENANT DE L'ACIDE PROPIONIQUE ET UN ESTER D'ACIDE FUMARIQUE

(30) Priorität: 19.10.2015 WO PCT/EP2015/074179; 24.02.2016 DE 102016103242
(43) Veröffentlichungstag der Anmeldung: 29.08.2018
(73) Patentinhaber: Flexopharm Brain GmbH & Co. KG, 44628 Herne (DE)
(72) Erfinder: GOLD, Ralf, 44799 Bochum (DE); HAGHIKIA, Aiden, 44789 Bochum (DE); MATTHES, Ulrich, 44623 Herne (DE)
(74) Vertreter: Schneiders & Behrendt Bochum
(86) Internationale Anmeldenummer: PCT/EP2016/066350
(87) Internationale Veröffentlichungsnummer: WO 2017/067681

(56) Entgegenhaltungen:
- WO-A1-2010/105112
- BERG JOHANNES ET AL: "Beneficial effects of short chain fatty acids on the course of experimental autoimmune encephalomyelitis", JOURNAL OF NEUROIMMUNOLOGY, Bd. 275, Nr. 1, 15. Oktober 2014 (2014-10-15), Seite 59, XP029083324, ISSN: 0165-5728, DOI: 10.1016/J.JNEUROIM.2014.08.154
- PATRICK M SMITH ET AL: "The Microbial Metabolites, Short-Chain Fatty Acids, Regulate Colonic Treg Cell Homeostasis", SCIENCE, Bd. 341, Nr. 6145, 2. August 2013 (2013-08-02), Seiten 569-573, XP055247108, US ISSN: 0036-8075, DOI: 10.1126/science.1237947
- YUKIHIRO FURUSAWA ET AL: "Commensal microbe-derived butyrate induces the differentiation of colonic regulatory T cells", NATURE, Bd. 504, Nr. 7480, 13. November 2013 (2013-11-13), Seiten 446-450, XP055178312, ISSN: 0028-0836, DOI: 10.1038/nature12721
- CATHARINA C. GROSS ET AL: "Dimethyl fumarate treatment alters circulating T helper cell subsets in multiple sclerosis", NEUROLOGY - NEUROIMMUNOLOGY NEUROINFLAMMATION, Bd. 3, Nr. 1, 10. Dezember 2015 (2015-12-10), Seite e183, XP055312762, DOI: 10.1212/NXI.0000000000000183
- A Haghikia ET AL: "Impact of fatty acids on CNS autoimmunity and their therapeutic potential for multiple sclerosis", , 7. Oktober 2015 (2015-10-07), Seiten 2-2, XP055247643, Gefunden im Internet: URL:http://citenpl.internal.epo.org/wf/web /citenpl/citenpl.html [gefunden am 2016-02-04]
- AIDEN HAGHIKIA ET AL: "Session 042 -MS and CNS Inflammatory Disease: Risk Factors for Disease Progression: P1.374 -Role of Fatty Acids in Multipe Sclerosis: Therapeutic Potential of Propionic Acid", AAN MEETING 2016, 16. April 2016 (2016-04-16), Seiten 1-1, XP055312758,
- DUSCHA ALEXANDER ET AL: "Propionic Acid Shapes the Multiple Sclerosis Disease Course by an Immunomodulatory Mechanism", CELL, ELSEVIER, AMSTERDAM, NL, vol. 180, no. 6, 10 March 2020 (2020-03-10), page 1067, XP086100512, ISSN: 0092-8674, DOI: 10.1016/J.CELL.2020.02.035 [retrieved on 2020-03-10]
- Erin E Longbrake ET AL: "Conclusions:", MULTIPLE SCLEROSIS JOURNAL (MSJ), vol. 22, no. 8, 12 October 2015 (2015-10-12), pages 1061-1070, XP055685066, BASINGSTOKE, GB ISSN: 1352-4585, DOI: 10.1177/1352458515608961

## Beschreibung

Die Erfindung betrifft eine Kombination zur Anwendung bei der Behandlung von Multipler Sklerose, Psoriasis und rheumatoider Arthritis, enthaltend Propionsäure, ihre physiologisch verträglichen Salze und/oder Methyl- oder Ethylester einerseits und eine oder mehrere Fumarsäureester und/oder deren physiologisch verträgliche Salze andererseits..

Nachstehend werden die Wirkung von Propionaten bzw. Fumaraten auf immunmediierten Entzündungserkrankungen für sich erläutert. Die Erfindung selbst richtet sich auf die oben beschriebene Kombination der Wirkstoffe.

Während bei Autoimmunerkrankungen körpereigene Strukturen Ziel einer fehlgesteuerten Immunantwort sind, beispielsweise bei Multipler Sklerose (MS), kommt es bei immunmediierten chronischen Entzündungserkrankungen zu Entzündungen verschiedener Gewebe, wie etwa am Darm (Morbus Crohn, Colitis ulcerosa), der Haut (Schuppenflechte) oder den Gelenken (rheumatischer Formenkreis). Allen genannten Krankheitszuständen ist gemeinsam, dass in der Folge der Entzündungen weitere Erkrankungen überdurchschnittlich häufig auftreten können, wie Übergewicht, Bluthochdruck, Arteriosklerose, Herzinfarkt und Schlaganfall.

Jüngste Erkenntnisse auf dem Gebiet der Mikrobiome haben gezeigt, dass Ernährung, das intestinale Mikrobiom und die lokale zelluläre Immunantwort miteinander verknüpft sind. Dies legt nahe, dass mit diätetischen Maßnahmen Einfluss auf die zelluläre Immunantwort und damit auf den Verlauf von Autoimmunerkrankungen sowie immunmediierten chronischen Entzündungserkrankungen genommen werden kann

Eine wesentliche Rolle spielt dabei die Diversität des Mikrobioms des Darms. Trotz vieler offener Fragen, insbesondere welche Komponenten des Mikrobioms tatsächlich für eine differenzierte adaptive Immunantwort im Darm verantwortlich sind, ergeben sich zunehmend empirische Hinweise darauf, dass einzelne Bakterienarten und ihre bakteriellen Metabolite einen starken Einfluss auf die systemische Immunantwort Zusammenhang mit Erkrankungen und Autoimmunität und immunmediierten chronischen Entzündungserkrankungen haben, beispielsweise für den Diabetes Typ 1und den Morbus Crohn.

Es hat sich ferner gezeigt, dass das Mikrobiom des Darms durch die Art der Nahrung beeinflusst werden kann und in der Lage ist, sich an die Anforderungen der jeweiligen Nahrung anzupassen. Dies bedeutet, dass eine für den Immunstatus eines Patienten ungünstige Darmflora durch diätetische Maßnahmen dahingehend geändert werden kann, dass sich der Immunstatus des Patienten verbessert.

Das Mikrobiom des Darms wie auch Ernährungsgewohnheiten, etwa hohe Salzaufnahme, wurden kürzlich als Umwelteinflüsse auf die Pathogenese von Multipler Sklerose (MS) als Prototyp einer von T-Zellen vermittelten Autoimmunerkrankung des zentralen Nervensystems identifiziert. Der Einfluss des Darm-Mikrobioms auf chronisch entzündliche Darmerkrankungen und Diabetes Typ 1 wurde bereits erwähnt. Auch bei Patienten, die an Diabetes Typ 2 leiden, wurden Besonderheiten in der Darmflora festgestellt. Inzwischen wird auch ein Zusammenhang mit neurodegenerativen Erkrankungen (Parkinson, Alzheimer) angenommen. In jüngster Zeit wurde ferner der Nachweis geführt, dass das Darm-Mikrobiom bei Niereninsuffizienz hochgradig verändert ist.

Eine zentrale Rolle spielen dabei regulatorische T-Zellen (Treg). Treg-Zellen, auch als Suppressor-T-Zellen bekannt, steuern die Aktivität des Immunsystems. Ein Mangel an Treg-Zellen wird mit zahlreichen Autoimmunerkrankungen in Verbindung gebracht.

Einen besonderen Einfluss auf das Mikrobiom im Darm und die regulatorischen T-Zellen haben Fettsäuren. Es wurde gefunden, dass langkettige Fettsäuren einen gegenläufigen pro-inflammatorischen Effekt bewirken. Überraschend ist, dass kurzkettige Fettsäuren eine positive Wirkung auf die Vermehrung und damit die Zahl der regulatorischen T-Zellen haben. Dies gilt insbesondere für Propionsäure, Buttersäure und deren physiologisch vertretbaren Salze und Ester. Es wurde ferner gezeigt, dass die gezielte Verabreichung kurzkettiger Fettsäuren mit drei bis sechs Kohlenstoffatomen einen positiven Einfluss auf das Entstehen und den Verlauf von neuroimmunologischen Erkrankungen mit neurodegenerativen Aspekten haben, wie beispielsweise MS.

Die Verwendung von Fumarsäureestern und -salzen, insbesondere von Dimethylfumarat und Salzen der Monomethylfumarsäure, zur Behandlung von MS und Schuppenflechte ist seit Jahren bekannt. Die Mittel sind gut wirksam, jedoch ist ihre Einnahme an der Wirkungsschwelle mit teilweise unangenehmen Nebenwirkungen verbunden, beispielsweise gastrointestinalen Problemen und einer Hautrötung. Die gastrointestinalen Probleme scheinen mit einer Reizung der Magenschleimhaut in Verbindung zu stehen. Zudem wurde die Einnahme von Fumaraten mit Leukopenien in Verbindung gebracht.

Fumarate werden - zumeist als Dimethylfumarat - in Tagesdosen von zumeist 2x 120 mg (initial) bzw. 2x 240 mg (Dauermedikation) verabreicht. In Einzelfällen kann eine höhere Dosis erforderlich sein. Die Patienten werden zumeist individuell eingestellt. Dabei wird in der MS-Therapie in der Regel Dimethylfumarat verabreicht, in der Psoriasistherapie Fumarate als Kombinationspräparat in Tagesdosen von 100 mg bis 1,2 g.

Aus A. Duscha et al., J. of Neuroimmunology, 3d. 275, 15.10.2014, S. 59 ist bekannt, dass Propionsäure bei der prophylaktischen Verabreichung an Mäuse den Verlauf einer experimentiellen Autoimmun-Encephalomyelitis abmildern kann.

Der Erfindung liegt die Erkenntnis zu Grunde, dass kurzkettige Fettsäuren geeignet sind, die Zahl der Treg-Zellen kurzfristig deutlich zu erhöhen, wenn sie in Verbindung mit Fumaraten verabreicht werden. Die Erhöhung ist bei Fumaraten sehr viel signifikanter als bei anderen für die Therapie von MS zugelassenen Medikamenten.

Entsprechend betrifft die Erfindung eine Kombination zur Anwendung bei der Behandlung vonMultipler Sklerose, Psoriasis und rheumatoider Arthtritis, enthaltend Propionsäure, , ihre physiologisch verträglichen Salze und/oder Methyl- oder Ethylester einerseits und einen oder mehrere Fumarsäureester und/oder deren physiologisch verträgliche Salze andererseits.

Der erfindungsgemäße Effekt ist auf Propionsäure, ihre physiologisch verträglichen Salze und/Oder Methyl- oder Ethylester beschränkt. Bei längerkettigen Carbonsäuren schlägt der Effekt ins Gegenteil um; Carbonsäuren mit zwölf oder mehr Kohlenstoffatomen zeigen in der Regel einen negativen Einfluss auf die Entwicklung und den Verlauf der Erkrankung.

Als physiologisch verträgliche Salze kommen in erster Linie die Alkali- und Erdalkalisalze in Frage, dazu Salze von physiologisch unbedenklichen oder essentiellen Schwermetallen, etwa Zink oder Eisen. Besonders bevorzugt sind bei den Alkalimetallen Natrium und Kalium und bei den Erdalkalisalzen Magnesium und Calcium.

Erfindungsgemäß anwendbare Ester sind die Methyl- und Ethylester.

Die Propionsäure beziehungsweise ihre Methyl- und/oder Ethylesterund Salze werden erfindungsgemäß mit Fumarsäureestern und deren Salzen kombiniert. Es versteht sich, dass die Wirkstoffe als Kombinationspräparat, aber auch getrennt verabreicht werden können.

Als Fumarsäureester kommt insbesondere Dimethylfumarat infrage, wobei aber auch andere Fumaratester mit C₁₋₆-Alkoholen eingesetzt werden können. Bevorzugt sind die Diester, jedoch können auch Salze des Monoesters eingesetzt werden, insbesondere die Alkali- und Erdalkalisalze sowie Zink- und Eisensalze. Neben den Natrium- und Kaliumsalzen sind insbesondere das Kalzium-, Magnesium- und Zinksalz bevorzugt.

Als Fumarsäurekomponente ist insbesondere auch eine Mischung aus Diemthylfumarat und den Kalzium-, Magnesium- und Zinksalzen von Ethylfumarat geeignet. Eine solche Mischung ist in der Dermatologie zur Behandlung von Schuppenflechte bekannt geworden.

Bei Propionsäure und ihren Salzen oder Estern beträgt eine maximale Tagesdosis bis zu 10 g vorzugsweise bis zu 5 g. In der Regel reichen Mengen von 0,5 g bis 1,5 g pro Tag aus, beispielsweise in Form von zwei Verabreichungen von 0,5 g morgens und abends. Eine Kapsel oder Tablette kann beispielsweise 0,5 g Natriumpropionat oder Kalziumpropionat enthalten.

Bei den Fumaraten beträgt die übliche Tagesdosis in der Regel 2x 120 mg oder 2x 240 mg in Tabletten- oder Kapselform. Bei der erfindungsgemäßen Kombinationen mit Fumaraten kann allerdings die maximale Tagesdosis gesenkt werden, etwa auf die Hälfte der oben angegebenen Werte. Dies vermindert die Nebenwirkungen.

Die erfindungsgemäße Kombination kann mit Vitaminen kombiniert werden, insbesondere mit Vitamin A, B12 und/oder D.

Unter Vitamin B12 werden erfindungsgemäß alle Verbindungen der Vitamin-B12-Gruppe verstanden, also Cobalamine und insbesondere Cyanolcobalamin und Speicherformen von Vitamin B12 sowie Coenzym B12.

Vitamin B12 hat sich als wirksam bei einer Reihe von Erkrankungen erwiesen, die mit Störungen des Immunsystems verbunden sind, etwa MS, Psoriasis und Neurodermitis. Es ist am Gesamtfettstoffwechsel beteiligt. Eine Fehlbesiedlung des Darms kann zu einem Vitamin B12-Mangel führen.

Eine Kombination der erfindungsgemäßen Kombination mit Vitamin B12 und vorzugsweise auch Vitamin D hat sich als vorteilhaft erwiesen. Vitamin B12 fördert insbesondere auch die Resorption von kurzfettigen Fettsäuren, etwa von Propionsäure, auch von Natriumpropionat.

Der Gehalt an Vitamin B12 orientiert sich an den empfohlenen Tagesdosen und liegt in der Regel bei maximal 5 µg pro Tag.

Das erfindungsgemäße Kombinationspräparat kann grundsätzlich in jeder marktfähigen Form vorliegen. Bevorzugt sind Kapseln und Tabletten. Die kurzkettigen Fettsäuren in fester Salzform, etwa als Natrium- oder Kalziumpropionat, können mit üblichen Tablettierungshilfsmitteln zu Tabletten gepresst werden.

Die einzelnen Wirkstoffe des erfindungsgemäßen Kombinationspräparats können gemeinsam, d.h. in einer Kapsel oder Tablette vorliegen, oder aber separat verkapselt oder tablettiert. Eine geeignete Form für die tägliche Verabreichung ist beispielsweise das Einbringen der Tagesdosis der Wirkstoffe, separat verkapselt, in einer Blisterpackung, etwa mit zwei Kapseln von Natrium- oder Kalziumpropionat à 0,5 g und einer Kapsel oder Tablette an Dimethylfumarat à 120 mg oder 240 mg. Etwaige Vitaminzusätze, insbesondere Vitamin B, werden in diesem Fall dem Fettsäuresalz zugemischt.

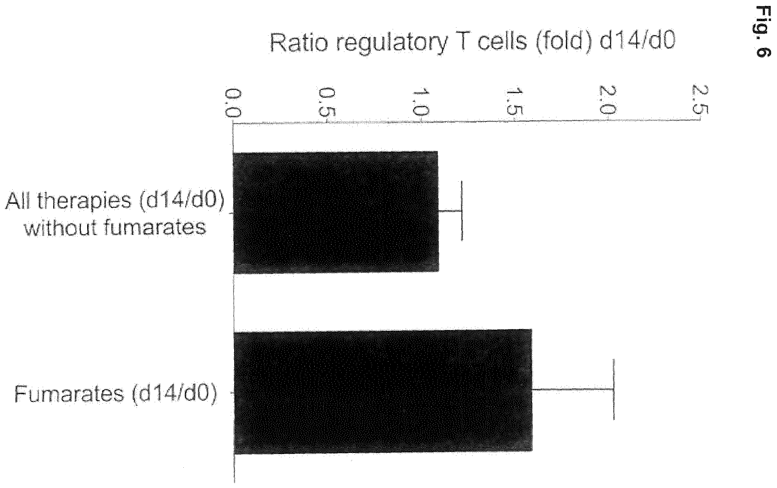

In Versuchen mit MS-Patienten konnte gezeigt werden, dass die kombinierte Verabreichung von Fumaraten (2x 120 oder 240 mg pro Tag) und Natriumpropionat (2x 0,5 g pro Tag) zu einer signifikanten Erhöhung der Treg-Zellen um den Faktor 1,6 führte. Im Vergleich dazu ergab sich bei anderen üblichen Mitteln zur Behandlung von MS zusammen mit Natriumpropionat nur eine Zunahme um den Faktor 1,1. Die überproportionale Steigerung der Treg-Zellenzahl steht für eine entsprechende Minderung der Autoimmunreaktion, siehe Fig. 6. Fumarate und Propionate wirken somit synergistisch zusammen. Das erfindungsgemäße Kombinationspräperat kann auch als Nahrungsergänzungsmittel eingesetzt werden.

Propionsäure hat Auswirkungen auf die Darmphysiologie und das dortige Mikrobiom. So nimmt sie Einfluss auf die Zusammensetzung des Mikrobioms. Die Anzahl an Propionat abbauenden Bakterien erhöht sich signifikant; der Einfluss auf die normale Darmflora bleibt dagegen gering. Dem gegenüber vermindern langkettige Carbonsäuren (Laurinsäure) die Zahl der Provotellaceae und einiger Familien von Bacteroidetes phylum bei Mäusen signifikant.

In der Fachwelt wird die Verbindung des metabolischen Syndroms mit entzündlichen Erkrankungen und insbesondere auch MS und Psoriasis diskutiert. Das metabolische Syndrom geht in der Regel einher mit einer Störung des intestinalen Mikrobioms. Propionat ist geeignet, derartigen Störungen entgegen zu wirken und Fehlentwicklungen zu beheben.

In mit Propionsäure behandelten Mäusen nimmt mit der Veränderung der Flora auch die Zahl der regulatorischen T-Zellen zu (CD4+ CD25+ Foxp3+ Treg). Genexpressionsanalysen von Signaturzytokinen zeigen erhöhte Werte für TGFβ1, IL-10 - im Allgemeinen anti-entzündliche Botenstoffe - und Foxp3 bei mit Propionsäure gefütterten Mäusen mit experimenteller autoimmuner Enzephalomyelitis (EAE).

Die aliphatische Kettenlänge der Carbonsäuren hat ferner eine Auswirkung auf die Th1/Th7-vermittelte Autoimmunität und die regulatorische Antwort der Treg im Mausmodell in vivo. Mäuse mit einer an Laurinsäure angereicherten Diät zeigten in EAE-Modell eine signifikante Reduktion der TH1- und TH17-Zellen im Dünndarm und gleichzeitig eine Ansammlung von Th1/Th17 im zentralen Nervensystem, was dafür spricht, dass die Steuerung der entzündlichen Zellen aus dem Darm ins Gehirn/Rückenmark gefördert wird. Propionsäure führte dagegen unter ansonsten gleichen Bedingungen zu einer deutlichen Zunahme von TGFβ1, IL-10 und Foxp3. Insgesamt ergab sich im Modell der MS bei Mäusen, die einer Diät mit langkettigen Fettsäuren unterworfen wurden, gegenüber einer Kontrollgruppe eine Verschlechterung des Zustands nach Einsetzen der induzierten Erkrankung, bei Mäusen die Propionsäure erhielten, bei prophylaktischer Anwendung, eine deutliche Verbesserung. MS und Psoriasis zeigen weithin Parallelen.

Im Ergebnis scheint Propionsäure in der Lage zu sein, ein gestörtes Gleichgewicht von Treg und Effektor-T-Zellen (Th1/Th17) zu ändern beziehungsweise zu normalisieren. Gerade Psoriasis-Patienten weisen ein solch gestörtes Gleichgewicht auf.

### Experimentelles

### (nicht zur Erfindung gehörend)

Unter standardisierten Bedingungen gehaltene Mäuse wurden normal, mit einer an langkettigen Fettsäuren (30,9%. Laurinsäure) angereicherten Diät und 200 µl täglich oral verabreichten Propionat ernährt. Das Propionat wurde entweder zum Zeitpunkt der Induktion der Erkrankung (DI) oder bei Eintritt der Erkrankung (OD) verabreicht.

Zur Induktion der EAE wurden die Mäuse anästhetisiert und mit insgesamt 200 µg MOG35-55 und 200 µg Freund Adjuvans (CFA) mit 4 mg/ml M. Tuberkulosis in zwei Subkutaninjektionen von 50 µl Emulsion links und rechts der Schwanzbasis behandelt. Pertussistoxin (200 mg/Maus) wurde intraperitoneal an den Tagen 0 und 2 nach der Induktion verabreicht. Die klinische Bewertung wurde täglich nach einer 5 Punkte-Skala (SEM) vorgenommen. Die Bewertung war wie folgt:
0 = normal
1 = Schwanzlähmung, beeinträchtigt das Aufrichten
2 = Gangataxie
3 = Paraparese der Hinterbeine
4 = Tetraparese
5 = Tod

Mäuse mit einem SEM von 4 oder 5 wurden ausgeschlossen.

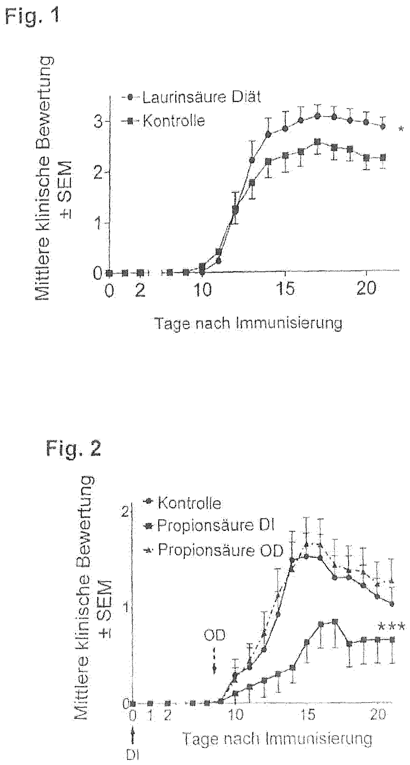

Die Ergebnisse sind in den Abbildungen 1 bis 5 dargestellt. Sie erläutern die Erfindung, sind aber nicht Teil der Erfindung.
Fig. 1 zeigt eine Auflistung der Mäusepopulation, die mit einer an Laurinsäure reichen Diät gefüttert wurden gegenüber einer Kontrollgruppe. Die Erkrankung setzte nach etwa zehn Tagen ein und erreichte nach siebzehn Tagen ihren Höhepunkt. Die Kontrollgruppe schnitt hinsichtlich der SEM-Werte besser ab als die Gruppe mit der an Laurinsäure reichen Diät.
Fig. 2 zeigt ein Diagramm, in dem die mit Propionsäure gefütterte Mäusepopulation einer Kontrollgruppe gegenüber gestellt wurde. Dabei wurde die Propionsäure entweder am Tag der Immunisierung (DI) oder am Tag des Auftretens der Symptome (OD) verabreicht. Es zeigte sich, dass die Gruppe, der die Propionsäure am Tage der Erkrankung verabreicht wurde, einen signifikanten günstigeren Verlauf der Erkrankung aufwies, als die Kontrollgruppe.

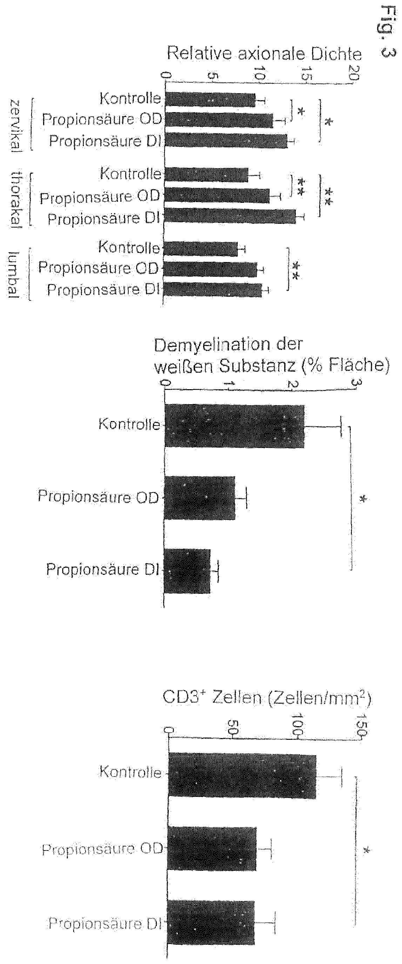

Fig. 3 zeigt den Einfluss von Propionsäure auf die relative axionale Dichte, die Demyelination der weißen Substanz und die Zahl der CD3+-Zellen. In jedem Fall ergibt sich durch die Verabreichung von Propionsäure eine deutliche Verbesserung des Zustands gegenüber der Kontrollgruppe für Propionsäure.

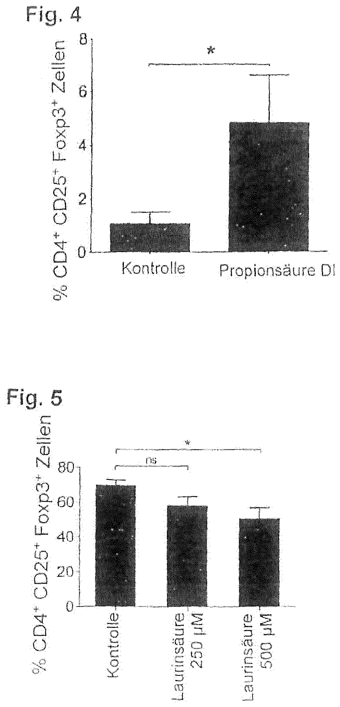

Fig. 4 zeigt den Einfluss der Verabreichung von Propionsäure auf die CD4+-CD25+ Foxp3-Zellen mit einer signifikanten Erhöhung gegenüber der Kontrollgruppe.
Fig. 5 zeigt den Einfluss der an Laurinsäure reichen Diät auf die CD4+ CD25+ Foxp3-Zellen im Vergleich zu einer Kontrollgruppe. Die Verabreichung der langkettigen Fettsäure führt gegenüber dem Vergleichswert zu einer Verminderung der T-Zellen. Die Verminderung ist abhängig von der Konzentration der langkettigen Fettsäure.
Fig. 6 zeigt eine Gegenüberstellung des Verhältnisses der Treg-Zellen nach Verabreichung von 2x 0,5 g Natriumpropionat zusammen mit einem eingeführten Mittel zur Behandlung von MS in der üblichen Dosis vor und nach 14-tägiger Behandlung.

Für die erfindungsgemäße Kombination von Natriumpropionat und Dimethylfumarat (2x 120 oder 240 mg täglich) ergab sich eine Steigerung der Zahl der Treg-Zellen um den Faktor 1,6. In einer Vergleichsgruppe, die mit der patientenspezifischen Dosis Interferon beta (Betaferon, Rebif 22), Glatirameracetat, Teriflunomid oder Fingolimod zusammen mit der oben angegebenen Menge Natriumpropionat behandelt wurde, stieg die Zahl der Treg-Zellen noch um das 1,1-fache.

## Patentansprüche

1. Kombination zur Anwendung bei der Behandlung von multipler Sklerose, Psoriasis und rheumatoider Arthritis, enthaltend Propionsäure, ihre physiologisch verträglichen Salze und/oder Methyl- oder Ethylester einerseits und einen oder mehrere Fumarsäureester und/oder deren physiologisch verträgliche Salze andererseits.

2. Kombination zur Anwendung nach Anspruch 1, **dadurch gekennzeichnet, dass** die Fumarsäureester Fumarsäuredimethylester und/oder die Salze von Fumarsäuremonomethylester sind.

3. Kombination zur Anwendung nach einem der Ansprüche 1 und 2, **dadurch gekennzeichnet, dass** sie eine Mischung aus Fumarsäuredimethylester und Salzen von Fumarsäuremonomethylester enthält.

4. Kombination zur Anwendung nach einem der vorstehenden Ansprüche, **dadurch gekennzeichnet, dass** die physiologisch vertretbaren Salze Natrium-, Kalium-, Magnesium-, Calcium-, Zink- und/oder Eisensalze sind.

5. Kombination zur Anwendung nach einem der vorstehenden Ansprüche, **dadurch gekennzeichnet, dass** sie Calcium- oder Natriumpropionat enthält.

6. Kombination zur Anwendung nach einem der vorstehenden Ansprüche, **dadurch gekennzeichnet, dass** sie zusätzlich Vitamin B12 enthält.

7. Kombination zur Anwendung nach einem der Ansprüche 1 bis 6, enthaltend die Wirkstoffe in einer Kapsel.

## Claims

1. Combination for use in the treatment of multiple sclerosis, psoriasis and rheumatoid arthritis containing propionic acid, its physiologically tolerated salts and/or methyl or ethyl esters on the one hand and one or more fumaric acid esters and/or their physiologically tolerated salts on the other hand.

2. Combination according to claim 1, **characterized in that** the fumaric acid esters are fumaric acid dimethyl ester and/or the salts of fumaric acid monomethyl ester.

3. Combination according to any one of claims 1 to 2, **characterized in that** said combination contains a mixture of fumaric acid dimethyl ester and salts of fumaric acid monomethyl ester.

4. Combination according to any one of the preceding claims, **characterized in that** the physiologically tolerable salts are salts of sodium, potassium, magnesium, calcium, zinc and/or iron.

5. Combination according to any one of the preceding claims, **characterized in that** said combination contains calcium or sodium propionate.

6. Combination according to any one of the preceding claims, **characterized in that** said combination additionally contains vitamin B12.

7. Combination according to any one of claims 1 to 6, containing the active ingredients in one capsule.

## Revendications

1. Combinaison pour utilisation dans le traitement de la sclérose en plaques, du psoriasis et de la polyarthrite rhumatoïde, contenant d'une part de l'acide propionique, ses sels et/ou esters méthyliques ou éthyliques physiologiquement compatibles et d'autre part un ou plusieurs esters de l'acide fumarique et/ou leurs sels physiologiquement compatibles.

2. Combinaison pour utilisation selon la revendication 1, **caractérisée en ce que** les esters de l'acide fumarique sont des esters diméthyliques de l'acide fumarique et/ou les sels d'esters monométhyliques de l'acide fumarique.

3. Combinaison pour utilisation selon l'une quelconque des revendications 1 et 2, **caractérisée en ce qu'**elle contient un mélange d'esters diméthyliques de l'acide fumarique et de sels d'esters monométhyliques de l'acide fumarique.

4. Combinaison pour utilisation selon l'une quelconque des revendications précédentes, **caractérisée en ce que** les sels physiologiquement acceptables sont des sels de sodium, de potassium, de magnésium, de calcium, de zinc et/ou de fer.

5. Combinaison pour utilisation selon l'une quelconque des revendications précédentes, **caractérisée en ce qu'**elle contient du propionate de calcium ou de sodium.

6. Combinaison pour utilisation selon l'une quelconque des revendications précédentes, **caractérisée en ce qu'**elle contient en outre de la vitamine B12.

7. Combinaison pour utilisation selon l'une quelconque des revendications 1 à 6, contenant les principes actifs dans une capsule.
